# EUROPEAN PATENT SPECIFICATION

(11) **EP 3 155 421 B1**
(45) Date of publication and mention of the grant of the patent: **20.02.2019**
(21) Application number: 15736631.1
(22) Date of filing: 11.06.2015
(51) Int. Cl.: G01N 33/493, G01N 31/22, B01L 3/00

(54) **CONTAINER FOR DETERMINING OF ANALYTES AND/OR CHEMICO-PHYSICAL PARAMETERS, AS WELL AS DETERMINING OF URINARY SEDIMENT, IN URINE; AND METHOD OF FULL URINE ANALYSIS USING THIS CONTAINER**
BEHÄLTER ZUR BESTIMMUNG VON ANALYTEN UND/ODER CHEMIKOPHYSIKALISCHEN PARAMETERN SOWIE ZUR BESTIMMUNG VON HARNSEDIMENT IN URIN UND VERFAHREN ZUR KOMPLETTURINANALYSE MITHILFE DIESES BEHÄLTERS
RÉCIPIENT POUR DÉTERMINER DES ANALYTES ET/OU DES PARAMÈTRES PHYSICO-CHIMIQUES, AINSI QUE POUR DÉTERMINER LE SÉDIMENT URINAIRE DANS L'URINE, ET PROCÉDÉ D'ANALYSE D'URINE COMPLÈTE À L'AIDE DE CE RÉCIPIENT

(30) Priority: 12.06.2014 IT RM20140308
(43) Date of publication of application: 19.04.2017
(73) Proprietor: Sikeliup S.r.l., 90128 Palermo (IT)
(72) Inventor: SPEZIALE, Danila Giuseppa Maria, I-92019 Sciacca (AG) (IT)
(74) Representative: Romano, Giuseppe
(86) International application number: PCT/IB2015/054423
(87) International publication number: WO 2015/189798

(56) References cited:
- EP-A1- 2 690 443
- WO-A2-2011/071364
- CN-U- 203 616 326
- US-A- 4 473 530
- US-A1- 2007 259 442
- US-A1- 2013 068 649
- Darci R. Block ET AL: "Automated urinalysis in the clinical lab", Medical Laboratory Observer Online, 1 October 2012 (2012-10-01), XP055209376, Retrieved from the Internet: URL:http://www.mlo-online.com/articles/201 210/automated-urinalysis-in-the-clinical-l ab.php [retrieved on 2015-08-25]
- J Geisel: "Automation in urinalysis including autovalidation with SIS-U", Sysmex Xtra Online, Vol.2, 1 December 2007 (2007-12-01), XP055209381, Retrieved from the Internet: URL:http://ru.sysmex-europe.com/files/arti cles/Xtra_UrinalysisAutomation.pdf [retrieved on 2015-08-25]

## Description

### Application field

The present invention relates to a container for the collection and standard analysis, not-microbiological, of urine samples, in order to measure the related chemical-physical parameters and the features of urinary sediment, for optimal use with automatic analyzers, supplying their operation with relevant advantages, in terms of management, economic and health implementation.

### Background

Today as in the past, the standard urine test is of primary importance in the diagnosis and evaluation of many clinical conditions, related not only to the urinary tract, but also to systemic diseases.

Running full examination of the urine, like in many other operating segments of modern Laboratory Medicine, has seen over the years the increasing of operational automation, so that, laboratories with medium or high degree of activity cannot leave anymore out of consideration a appropriate and efficient level of automation, to adequately supply the high number of daily samples and the needed requirements to continuously provide in a short time reliable results and high operational quality.

For this reason, many Companies, operating in this specific field, put their efforts in producing and providing analyzers, mostly integrated, able to process a high number of samples/hour, providing in a short time the results of the chemical-physical and urinary sediment test, directly in connection with LIS (Laboratory Information System), and leaving to specialist, both in laboratory and clinical field, only the necessary and appropriate assessments related to pathological case and/or to diagnostic questions.

The definitions of physical-chemical examination and urine sediment, refer to two different analytical evaluation, each one characterized by finding of very specific parameters related to different analytes and/or to characteristics of the urine sample.

The **urine physical-chemical examination** by an automatic analyzer provides for the determination of an given number of parameters (usually ten) that will be listed below, using refractometric and light-scattering methodologies.

The **examination of the urinary sediment** takes over both endogenous cellularity of the organism present in the urinary excretion (white blood cells, red blood cells, skin cells) and the exogenous one (bacterial microorganisms, and less commonly fungal or parasitic microorganisms), besides the presence of crystals or other formations visible at the optical microscope, providing for each sample overall numerical results and/or per microscopic field and/or per microliter.

Currently, running automatic mode, the execution of the standard and full urine examination is realized in two distinct and separate stages, each one operationally managed by a specific analyzer, whose related hardware devices can be kept separate or incorporated into a single instrument.

The routine urine examination through the use of simple multiparametric strips, represents the first diagnostic step for a large number of diseases. The common diagnostic strip for urine examination is made by a plastic or paper ribbon, having a width usually not more than five mm, on which reaction pads are coated, using appropriate substrates able to detect specific substances present in urine, through a chemical colorimetric reaction. In this way, by contact with the urinary material, the simultaneous detection of urinary parameters of clinical-diagnostic interest is achieved.

In the market there are various types of strips: a qualitative one, leading to a result expressed only as Negative/Positive, and a semiquantitative type, adding an estimate of the concentration of the detected analyte/parameter. In the latter case, the color reaction of the substrate is proportional to its concentration, when compared to a specific reference range.

The standard type of urine diagnostic strip generally includes ten reaction pads, each of them using a specific chemical reaction in highlighting the urine sample characteristics.

The measured parameters are generally represented by: Color, Turbidity, PH, Specific Gravity, Protein, Glucose, Bilirubin, Urobilinogen, Ketone bodies, Hemoglobin, Nitrite and Leukocytes.

The contact between the substrates of the strip and of the urine sample is achieved by immersion, usually lasting a few seconds, and the next reading time of the reactions is between 60" and 120". Exceeding these values, the reactions cannot be properly interpreted.

In case a manual methodology is adopted, the highlighted color is compared by the operator performing the examination, with a reference color scale for each specific analyte.

In the latter case, the procedure includes the following operation steps performed by the operator:
1. The strip is completely immersed for a few seconds in the urine specimen, properly mixed;
2. The strip is subsequently extracted, and every excess of urine leftover is removed;
3. Must be ensured that the contact time between urine and reaction pads of the strip, is generally between 60 "and 120".
4. The generated color is visually compared to a chromatic scale specific for each analyte, usually made available by the supplier, along with the package containing the strips.

Incorrect or inadequate operations, easily leads to analytical errors, among which the most common are:
- Underestimation of the presence of erythrocytes and leukocytes in case the sample is not properly mixed prior to immersion of the strip, as these cellular elements tend to fall by gravity to the bottom of the test tube.
- Failure on removing the excess of urine from the strip, and then from the reaction pads, can produce the spreading of urine to a closer pad, and hence to a color alteration which leads to an inaccurate result. For this reason, it is recommended the application of absorbing paper on the edge of the strip in order to remove excess urine.

Using automatic analyzers, small amounts of urine are taken from the original urine samples positioned on suitable sample-holder racks, using a proper sampler needle, and then distributed onto the reactive substrates of the strips previously placed inside the analyzer. The colorimetric reading of the reaction pad placed on the strip is carried out by an automatic reader with LED technology, and the results provided in internationally validated Units. The readings of the color changes, depending on the nature and amount of the analyte/parameter found, occur at certain wavelengths, according to predetermined chromatic scales. The results are expressed in a qualitative or semi-quantitative way.

At present, in automatic mode and for the determination of chemical-physical properties above described, it is necessary the presence of hardware and software dedicated to this purpose, operatively connected with other different instrumentation, also both hardware and software, suited to perform the examination of the urinary sediment, as above specified.

Nowadays, various types of containers are available for the use in the Laboratory Diagnostics, able to collect urine samples and determine within the same container various parameters and/or analytes.

Most of them provides for the execution of screening tests for the detection of urinary catabolites, arising from the assumption of so-called drugs of abuse. Their increasing use makes necessary for the sports, government and health structures or organizations, the application of more and more tight programs in order to control and prevent the rising spread of this phenomenon, in addition to obvious security reasons at the workplace and in terms of public order, especially in case of criminal events and/or road accidents. Such devices, for this reasons, contain several sealing systems and security measures, aimed at avoiding accidental spills or tampering of the urine sample, such as their replacing and adulteration, in some cases with both visual and acoustic indications of imperfect closure of the lid.

In these cases, given the specific typology of the sought substances, the used analytical methodology is not colorimetric (as it is, on the contrary, provided by this invention), but uses the classic principles of a immunological test (immunoassay), in which small amounts of urine sample, imbibe by capillarity the reactive strip, causing the formation of a antigen/antibody complex and the presence or absence of a linear reaction band in "*Test*" position, depending on, whether or not, the method exploits a competitive binding, along with the specific control band of the reaction. Information given from this type of test is only qualitative, since it provides a negativity/positivity result only by the evaluation of an operator, who can possibly store the image itself. The evaluation of the results by the operator must be necessarily done within a predetermined time range, different from that provided for the colorimetric reactions, and generally comprised between 5 and 10 minutes, starting from the moment in which the sample is absorbed by the reactive strip: otherwise the result of the test is to be considered uninterpretable. In any case, the urine left over after the test can never be processed subsequently for the examination of the urinary sediment (as it is on the contrary provided by the present invention), but only for a potential subsequent confirmation tests related only to the consumption of drugs of abuse.

The strip and the corresponding analyte under investigation are identified with a simple alphabetical code corresponding to the metabolite of the sought substance, to be used only by the operator, during the manual reading of the detected analyte.

In the event that the field of interest is represented by the determination of chemical-physical parameters and urinary sediment, almost all existing containers need the withdrawal of a certain amount of the urine sample outside the container used for the collection: in this way the evaluation of the parameters is only performed outside of the tube, both in manual and automatic mode. In the latter case, this occurs by the transfer of predetermined amounts of urine through dispensing devices, with subsequent reading of the reactions on the strip previously handled and positioned within the automatic instrument.

However, even when a container for urine collection with a reactive strip assembled inside is proposed, the operating procedures associated need the manual transfer of the sample inside it after the initial collection of the urine into a primary tube, thus having an open system, and only a manual mode of reading the results by the operator. The urine residue remains available for reading of the urinary sediment only if this is performed always in the manual mode using a Optical Microscope.

In any case, no urine container, among those already known, allows the full examination of the physical and chemical parameters together with the sediment by an automatic analyzer, either because completely unsuitable for that purpose or, in the case of standard containers of common use, only on condition that predetermined amounts of urine are properly placed inside the instrument and analyzed in compartments not fixed to the container itself.

WO2011/071364 discloses a container for drugs detection, having an outer container and an inner container. The inner container act as containment region, the
outer container act as detection/reading region. The passage of the urine from the inner container into the outer container is obtained by breaking a septum, the breaking occurring when the container is closed by means of its screw cap. Pressure of the cap on the inner container forces the latter against a tip which breaks the septum and allows the urine to flow towards the detection/reading region.

Using the object of the present invention, on the contrary, the automatic processing of chemical and physical parameters of the urinary sample happens in a analytical region inside the structure of the container itself, whose results are made effectively available to the evaluation by the analyzer, without involving him in the aforesaid step of processing the sample on reactive strips outside.

### Brief description of the invention

The purpose of the present invention is to improve the analytical operation of the standard examination of the urine in automatic mode, but also in case of a manual method, overcoming problems and issues above explained, and this is achieved both through the adoption a container, which incorporates in the structure itself, the individual reaction pads able to detect the parameters previously specified, as defined in claim 1, as well as through the original procedural steps better described below and defined in claim n. 12.

Further characteristics of the present invention are defined in the corresponding dependent claims.

The present invention, as compared to the operational characteristics of the known procedures, defines numerous and obvious advantages, making more functional the multi-parametric analysis of chemical and physical parameters involved in urinary diagnostics.

In particular, the known procedure shows that: in *manual mode*, the reaction occurs as a result of immersion of the multi-parametric strip in the urinary liquid, with simultaneous, not standardized contact to each reaction pad; in *automatic mode* dispensing of urine on the strip is automated through calibrated tips. Both in manual and in the automatic mode, the multiparametric strip are not integral with the standard container collecting the urine sample under examination.

The present invention provides individual single-reaction pads, integrated within and unite to the device itself, that come in contact with standardized amounts of urine, showing specific colorimetric reactions according to the concentration of the detected analyte/parameter.

The position of each pad is not constrained to any specific orientation, but only to the space available inside the device, in any way remaining unaltered the mode of imbibition of the reactive substrate.

The imbibition of the pad occurs by direct contact with the urine sample, on a reaction surface appropriately calibrated, in order to avoid artifacts related to contact with excessive amounts of urine.

Moreover, by the assembly and use of single-reaction pads, the container according to the present invention, doesn't require any manual intervention by healthcare operators. In case the device is used in manual mode, the only required operator intervention, but still in a closed system and without any spillage of urine outside, provides for the piercing of the septum inside the device itself, through suitable closure cap. Moreover, the device of the present invention advantageously provides for a system of vacuum suction, so as to collect the proper amount of urine in the closed mode and without the possibility of contamination outside.

The configuration of the device in terms of structured container implies:
i) the possibility to use the container on analytical fully automated instrumentation, thus making possible the execution of a standard and complete urine examination,
ii) the possibility of not having to use a more substantial part of the hardware and software currently equipping the modern automatic analyzers,
iii) the ability to speed up the operating routine,
iv) the elimination of problems related to the housing and handling of reactive strip inside the automatic analyzer, as well as their separate disposal.

In particular, unlike the known procedure, the present invention is typically suitable to be used on an automated analyzer because of the peculiar distinctive characteristics listed below:
a) standard dimensions of the container allowing to be housed on the sample-holder racks used by routine on the automatic analyzers;
b) the presence of pre-calibrated vacuum inside the device, proper to recall the correct and optimal amount of urine coming from the primary container of sample collection;
c) presence of pierceable and recloseable closure, suitable to sampling by needle/tip in automated mode;
d) guarantee of the appropriate reaction time of the urinary sample and colorimetric reaction pads, standardized, operator-independent, and controlled by definition of innovative procedure using a separation septum inside;
e) exposure mode of reaction pad suitable for automated reading by integrated optical sensors;
f) identification of the reaction pad by bar codes, suitable for automated recognition by optical readers integrated in automated analyzers;
g) presence of spots to start and end automated reading, as well as signaling for technical errors and/or reading artifacts.

In particular, characteristic d) is absolutely necessary and fully meets the quality standards required in automatic analyzers.

This container eliminates the withdrawal of specific amounts of urine for external processing of these tests by automated analyzers, as well as the manual imbibition of reactive strips on the urinary sample. Moreover it makes unnecessary, in manual mode, the device opening, in order to allow the contact with conventional reactive strips, thus reducing the related risks of biological contamination.

One of the purpose of the invention is also to make no longer required the use of instrumentation, integrated or separate, dedicated to the physical-chemical analysis of urine, making only sufficient a LED reading of color reactions, that can be easily placed on the specific analyzer for the urinary sediment

The operational advantages of this device are therefore both economical and operational, given the improved productivity and easiness of use, the lowering of contamination degree of the analyzer, and the drastic reduction of costs associated with the production, supply, maintenance and service of a analyzers so structured.

### Detailed description of the invention

The advantages arising from the present invention, together with the characteristics and conditions of employment of the given device, will become clear from the following detailed description, relating to its embodiments, suitable to the purposes above mentioned, and presented for illustrative but not limitative purposes.

We will refer to the figures of the attached drawings, where:
- Figure 1 is a perspective view of a container according to an embodiment of the present invention;
- Figures 2A, 2B and 2C show, as example, possible variations of embodiments of the container according to the present invention;

- Figures 3A to 3D illustrate how to use a container according to further embodiments of the present invention;
- Figures 3E and 3F show a possible embodiment of a separation septum for a container according to the present invention;
- Figures 4A and 4B show a container according to a further embodiment of the present invention;
- Figures 5A and 5B show a container according to a further embodiment of the present invention;
- Figures 6A and 6B show, as example, possible forms of the reaction pad according to the present invention.

The present invention will be described below with reference to Figures above mentioned.

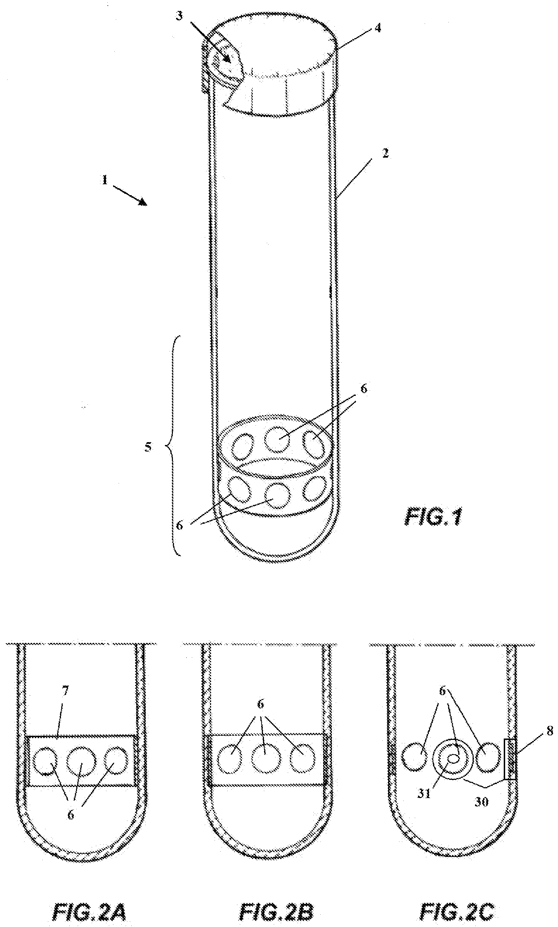

With reference to figure 1, this shows a container for urine samples according to one embodiment of the present invention.

For the purposes of this description, the container 1 will always be described as a test tube, thus having a body 2 with a tubular shape, elongated and with a main body including a containment region 9 of the urinary material, having an opening 3 at its upper end and an underlying reading region 5 in which are allocated one or more reaction pads.

The container includes a pre-calibrated vacuum in its interior, in correspondence of the containment region, so as to effectively respond to the usual safety requirements.

However, it has to be clarified that such a tubular shape of the test tube is not a limiting feature of the present invention. There may be in fact other forms responding to the same inventive concept.

The container includes a closure element 4 to seal in a removable manner the opening 3. The closure element 4 may be applied to the container through a screw system, pressure, or other equivalent system. The closure element 4 will have to be made by material pierceable by a sampler/mixer needle 20 and will have to ensure the tightness with the external environment even after the said needle 20 will be spilled from the element 4.

As already indicated, the container 1 according to the present invention provides a reading region 5. Such reading region 5 holds one or more reaction pads 6, made integral to an inner wall of the main body 2.

Each reaction pad is able to absorb the fluid and to result in a colorimetric reaction specifically for a respective analyte and/or measurement of interest.

One of the advantages of such a realization is that it is not longer necessary to pick up and carry various amounts of urine outside the device to perform the biochemical reaction on a specific automated analyzer.

For the realization of the reaction pad, and of the reactive components specific for the different analytes and/or parameters to be determined, see the following description.

The following Figures 2A to 2C illustrate several variants relating to the allocation mode of the reaction pads 6 on the inner wall of the container, in correspondence with the reading region.

In particular, the first possibility is illustrated in Figure 2A, in which the reaction pads (one or more of them) are based on a strip 7 which then is fixed to an inner wall of the main body, in correspondence with the reading region 5.

Advantageously, this strip 7 is made of non-absorbent material, so as not to interfere with the imbibition of the reaction pad. Moreover, it can advantageously be done in order to be opaque in the space between a pad and the other, so as to reduce or eliminate possible interferences during the colorimetric reading of the results.

In Figure 2B a further variant is shown, with the inner wall of the main body having a recess adapted to contain the area of the strip 7 along its entire thickness.

Figure 2C illustrates a further variant, with the inner wall of the main body presenting, for each reaction pad, a corresponding area of recess adapted to contain the pad for its entire thickness.

The advantage of the embodiments illustrated in Figure 2B and 2C (with particular reference to the presence of a recess) is constituted by a greater stability of placement of the reaction pad, imprisoned within a specific housing, in order to avoid any defects in adhesion and/or welding of the same in the test tube itself.

In all cases above described, it is advantageous that the areas of the main body between the distinct reaction pads are opaque, in order to reduce or eliminate possible interference during the colorimetric reading of the results.

According to a possible embodiment of the invention, to the rear of each individual pad of reaction, on the side facing the inside of the container, an insulating protection film 30 can advantageously be provided, having a calibrated hole 31 to allow contact with a correct urine amount, thus avoiding an excessive imbibition of the pad with the same urine.

One of the critical aspects in this type of analysis is definitely the reaction time, which, as already specified, is particularly short (60" - 120").

It follows that, in the known procedure, the urine collection should take place in suitable containers, and that the specific colorimetric reactions occur subsequently, or following manual insertion of the strips in the urine sample or following automatic withdrawal of small amounts of urine liquid that are distributed on other reaction elements, external to the device containing the sample.

According to an embodiment, the present invention addresses and solves this problem too.

In particular, the figures from 3A to 3D relate to this embodiment.

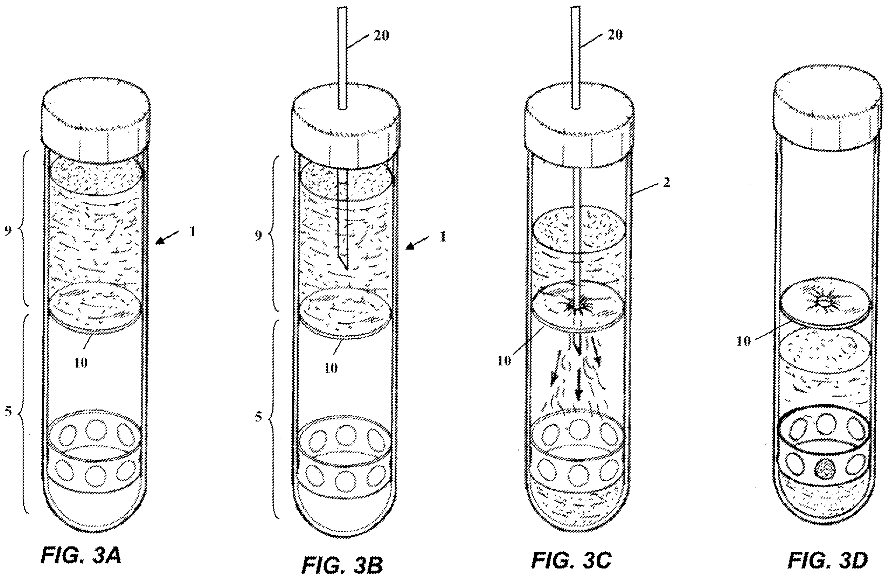

With reference to Figure 3A, the container 1 may include a separation septum 10, impermeable and pierceable, positioned between the containment region and the reading one.

The septum 10 is a separation surface able to divide into two separate compartments the inside part of the test tube itself: the collection or containment compartment 9 and the reading region 5.

The compartment 9 is designed for the initial collection of the urine to be analyzed, without this being in contact with the reaction pads which instead are located in the reading region 5.

Of course it is to be considered that the septum 10 will be made in suitable materials, as not to contaminate the possible biological matrix.

For example, the separation septum can be made entirely in the form of an aluminum sheet, easily drillable.

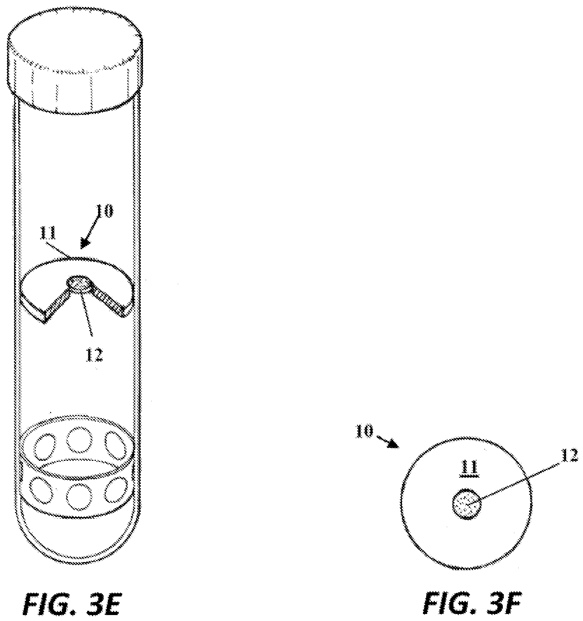

Alternatively, as visible in Figures 3E and 3F, the separation septum may comprise a peripheral region 11 of plastic material and a central region (12), made of aluminum and pierceable.

As illustrated in Figure 3B, in the use of the container 1 on an automatic analyzer, a sampler and mixer needle 20 can be used to perforate the closure element 4 and mix the urine present in the containment region 9.

Then, withdrawing via the sampler needle 20 a predetermined quantity of urine, this can be dedicated to reading of the urinary sediment.

Subsequently, as shown in Figure 3C, always through the sampler needle 20, the perforation of the septum 10 it can be carried out.

As illustrated in Figure 3B, the drilling of such a surface, within an analyzer, can take place e.g. by a needle sampler 20, or with any other equivalent device.

The drilling of the septum 10 then allows the urinary liquid to flow in the reading region of the test tube, to soak the reaction pad and therefore to result in the colorimetric reactions within the correct timing (Figure 3D).

As already indicated, it is not strictly necessary that the container according to the present invention presents in the form of a classic test tube.

In particular, the container may not have an exactly cylindrical shape for the whole or part of its height. For example, some portions of the wall may be flat, so as to enable the sensor of the analyzer for a better reading of the colorimetric reactions from the reaction pad.

Therefore, there may be provided, for example, shapes with different section for the test tube or for parts of it.

Based on the location of the reaction pad and the conformation of the tube, it may be provided for an automatic rotation of the tube itself during the reading of the analyzer.

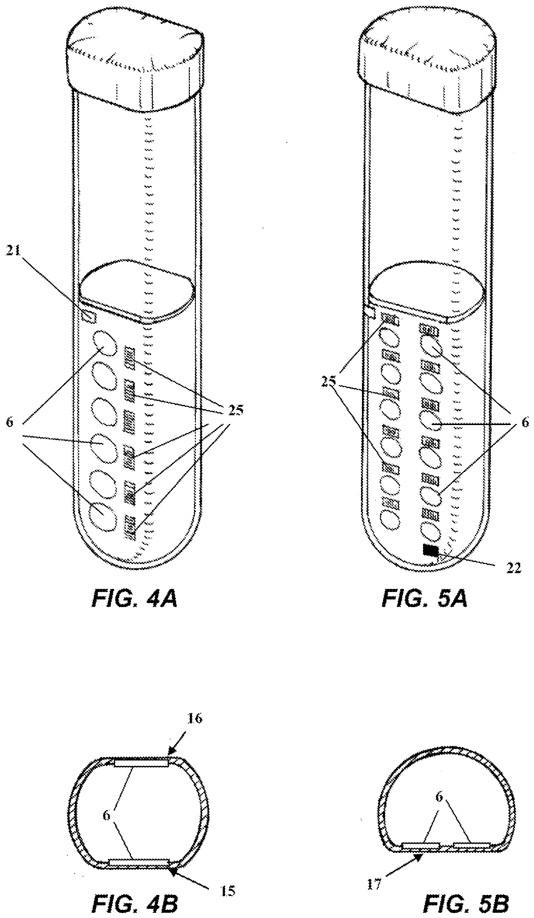

Figures 4A and 4B show, as example, a possible shape for the container according to the present invention.

In this case, the container has a tubular shape, in cross section, has two opposite flat faces 15, 16.

On each of these faces, the reaction pads 6 are placed, as so far described.

Figures 5A and 5B, however, relate to an embodiment according to the test tube with a single flat surface 17, inside which the reaction pads are placed, for example on two columns.

Advantageously, and for each of the embodiments described so far, each reaction pad is associated to an identification barcode not alphanumeric, related to the corresponding analyte determined by it.

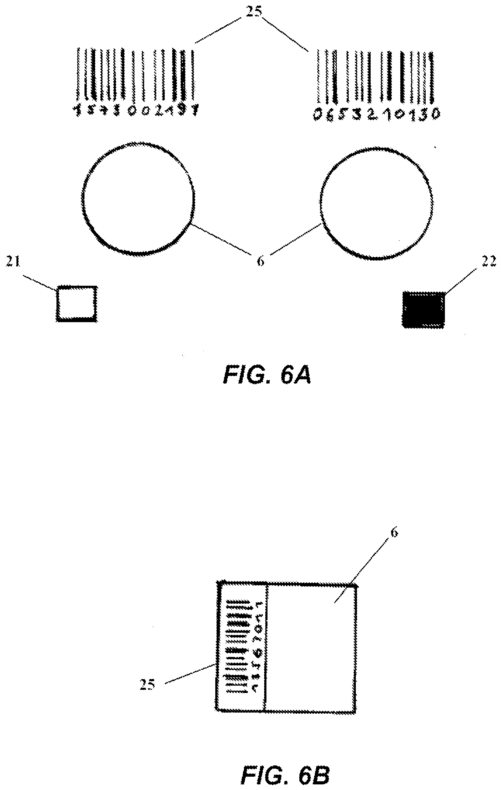

Figures 6A and 6B relate to the possible implementations of identification codes associated to the reaction pads.

In particular, each reagent pad may be marked, on the top or bottom side, with an identification code (e.g. barcode or QRcode) of the pad itself, and/or of the single reaction, that can be detected by automatic sensors.

The codes may be prepared in common assembly with each reaction pad or on adherent strip inside the test tube, so that each code matches a single reference pad.

It is to be considered that the shape of the reaction pad can be different (square, round, rectangular, etc..) depending on the specific embodiment of the container, so as to allow a better stability in the housing, and an optimal reading surface of the sensor.

As example, Figure 6B shows a square reaction pad, with an associated barcode right adjacent to one of the four sides.

Moreover, according to possible alternative embodiments, it can be envisaged that one or more of the reaction pads have the function of reactive control pads, to be used as reference measurement during the automatic reading.

This spot has the task of indicating, whereas the color change of the colorimetric range exceeds the maximum tolerated, the excessive prolongation of the contact time between the reaction pad and the urine, and therefore the automatic reading of the test tube (or the possible re-reading by subsequent passage on the analyzer) would be wrong, thus not being possible its validations.

Advantageously it can be inserted, on the inside part of the tube, even a spot capable of detecting the indices of interference with the optical reading.

Moreover, the codes 21, 22 ,indicating the start and end of the automatic reading, can be inserted.

For completeness, just as example, following there is a brief description of general chemical principles that determine the reactions between the used substrates and the main parameters tested in the urine:

### Bilirubin

The coupling of bilirubin with a diazonium salt in an acidic environment results in a red azo coloring agent.

In the range of colors the following values are reported: 0 (negative), 1 (+), 2 (++), 4 (+++) mg/dl or resp. 0 (negative), 17 (+), 35 (++), 70 (+++) µmol/l.

Bilirubin concentrations are reported starting from 0.5-1 mg/dl.

### Proteins

The test is based on the principle of the protein error of a pH indicator. The test is primarily reactive with albumin.

In the range of colors the following concentrations of albumin are reported: negative, 30, 100, and 500 mg/dl and resp. negative, 0.3, 1.0 and 5.0 g/l. The concentrations of albumin are reported starting from about 15 mg/dl.

### Blood (Haemoglobin)

In the presence of organic hydroperoxides and a chromogen, the peroxidase action of hemoglobin and myoglobin results in a green color. The intact erythrocytes are indicated by color spots in the reactive area, while the hemoglobin and myoglobin result in a uniform green color.

In the range of colors the following values are reported: 0 (negative), ca. 5-10, ca. 50, ca. 300 erythrocytes/µL.

The concentrations are reported starting from about 5 erythrocytes/µL.

### Glucose

The test is based on the specific reaction glucose-oxidase/peroxidase.

In the range of colors the following values are reported: normal, 50, 100, 250, 500 and 1000 mg/dl. Glucose concentrations are reported starting from 40 mg/dl.

### Ketone bodies

The acetoacetic acid and acetone react with sodium nitroprusside in alkaline solutions, resulting in a compound of purple coloring (Legal test).

In the range of colors the following acetoacetic acid concentrations are reported: 0 (negative), 25 (+), 100 (++) and 300 (+++) mg/dl resp. 0 (negative), 2.5 (+), 10 (++) and 30 (+++) µmol/l.

### Urobilinogen

The test is based on the reaction of urobilinogen with a diazonium stable salt that results in a red azo coloring agent.

In the range of colors the following concentrations of urobilinogen are reported: normal, 2, 4, 8, 12 mg/dl and resp. normal, 35, 70.140, 200 µmol/l.

### Leukocytes

The granulocyte esterases dissociate an ester of carbonic acid heterocyclic. The fragment reacts with a diazonium salt and results in a purple coloring.

In the range of colors the following values are reported: 0 (negative), ca. 25, ca. 75, ca. 500 leukocytes/µL. The concentrations are reported starting from 30 10-20 leukocytes/µL.

### Nitrites

The test is based on the principle of Griess reaction. Any pink coloring is to be interpreted as a positive result and indicates the presence of 105 organisms/ml of urine. The concentrations of nitrite are reported starting from 0.05-0.1 mg/dl.

### PH

The test contains an indicator of mixing, able to differentiate clearly, in the values of pH 5 to 9, a range of colors ranging from orange, to yellow and cyan.

In the range of colors the following pH values are reported: 5, 6, 7, 8, 9.

### Specific weight / density

The test is based on the color change of the reagent from blue-green to green-yellow, dependent on the concentration of ionic components in the urine. The test allows to determine the values of specific weight between 1.000 and 1.030.

In the range of colors the following concentrations are reported: 1.000, 1.005, 1,010, 1,020, 1,025 and 1,030.

Each of the above parameters corresponds to a different composition of the reaction pad.

The reactive components of the substrates, for the above parameters can therefore be the following:
- Ascorbic acid: 2,6-dichloro fenolindofenole (0.7%)
- Bilirubin: diazonium salt 3.1%
   - Blood: tetramethylbenzidine dihydrochloride 2.0%, isopropilbenzolo, hydroperoxide 21.0%
- Glucose: glucose-oxidase 2.1%, peroxidase 0.9%, o-tolidine hydrochloride 5%
- Ketone bodies: sodium nitroprusside 2%
- Leukocytes: carbonic acid ester 0.4%, diazonium salt 0.2%
   - Nitrites: tetraidrobenzochinolin-3-ol, 1.5%, sulfanilic acid 1.9%
- PH: methyl red 2.0%, bromothymol blue 10.0%
- Protein: bromophenol blue tetra 0.2%
- Specific Gravity: bromothymol blue 2.8%
- Urobilinogen: diazonium salt 3.6%

The present invention has hereto been described with reference to its preferred embodiments. It is to be mentioned that the technical solutions implemented in the embodiments described here as example, may advantageously be combined differently between them, to result to other embodiments afferent to the same inventive core and all however falling within the scope of protection of the claims hereafter reported.

In particular, the materials, methods and techniques used in the design and implementation of the present invention can be implemented by individual manufacturing companies based on their specific know-how, technology and operations, resulting in any case always included as part of what is expressed and claimed by the present invention.

## Claims

1. Container (1) apt to the running of a standard full urine test comprising the determination of analytes and/or chemico-physical parameters and of urinary sediment on urine via an automated analyzer in a closed mode, comprising:
a) a containment region (9) having an opening (3) with a tighten and pierceable and recloseable closure element (4), suitable to sampling by a needle in automated and closed mode;
b) a reading region (5) having one or more reaction pad (6) made integral to an inner wall of the container, each reaction pad (6) being apt to absorb a pre-established amount of urine and to give rise to a colorimetric reaction specific for a respective analyte, parameter and/or reference;
c) a separation septum (10), impermeable and pierceable, positioned between the said containment region (9) and the said reading region (5).

2. Container (1) according to claim 1, wherein the containment region (9) has a precalibrated vacuum suitable for the recall of a quantity of urine from a primary container of collected sample.

3. Container (1) according to claim 1, wherein said separation septum (10) is made, at least in part, of aluminum.

4. Container (1) according to claim 3, wherein said separation septum (10) comprises a peripheral region (11) in plastic material and a pierceable central region (12) in aluminum.

5. Container (1) according to one of the preceding claims, wherein said inner wall of the container has at least a recess zone apt to contain a respective pad (6) for its whole thickness.

6. Container (1) according to one of the preceding claims, wherein one or more of said pad (6) is made on a strip (7) fixed at an inner wall of said reading region (5).

7. Container (1) according to claim 6, wherein said inner wall of said reading region (5) has a recess zone apt to contain said strip (7) for its whole thickness.

8. Container (1) according to one of the preceding claims, wherein said containment region (9) and said reading region (5) have at least a portion of plane wall (15, 16, 17), said at least a portion of plane wall (15, 16, 17) configured to house said reaction pads (6).

9. Container (1) according to one of the preceding claims, wherein to each reaction pad (6) is associated a code (25) identifying a correspondent analyte and/or parameter, apt to be recognized by an optical reader integrated in the automated analyzer.

10. Container (1) according to one of the preceding claims, wherein the zones between different reaction pads (6) present in the reading region (5) are made opaque.

11. Container (1) according to one of the preceding claims, comprising a protective non-permeable film (30), made adherent at the back to said reaction pads (6) on their side faced to the inner wall of the container (1) in the reading region (5), and having a calibrated hole (31) at each of said pads (6) allowing the contact with a correct amount of urine.

12. Method to run a standard full test of urine on an automated analyzer, including physical-chemical examination and urine sediment on urine via an automated analyzer in a closed mode, which comprises the following procedural steps:
a) filling a container (1) according to any one of the preceding claims with a urine sample, from a primary collection container by suction regulated from pre-calibrated vacuum, in closed mode from the outside;
b) loading the container (1) on the automatic analyzer;
c) piercing the pierceable and reclosable closure element (4) with a sampler and mixer needle (20) so as to insert the needle (20) in the containment region (9) according to a closed mode;
d) mixing the sample present in the containment region (9) and withdraw in a closed mode a predetermined amount of urine;
e) transferring such predetermined amount to the analyzer for the automatic reading of urinary sediment;
f) piercing and the septum (10) and retract the sampler needle (20), in such a way that the urine flows in the reading region (5) coming into contact with the reaction pads (6);
g) waiting for a specified time for the generation of color reactions;
h) performing an automatic reading of the colorimetric reactions using a sensor integrated into the analyzer.

13. Method according to claim 12, wherein said reading step provides for a rotation of the container (1) along its longitudinal axis.

## Patentansprüche

1. Behälter (1), der geeignet ist, einen standardisierten vollständigen Urintest durchzuführen, welcher die Bestimmung von Analyten und/oder chemischphysikalischer Parameter sowie von Harnsediment von Urin über einen automatisierten Analysierer in einer geschlossenen Betriebsart umfasst, umfassend:
a) eine Behältnisregion (9) mit einer Öffnung (3) mit einem engen und durchdringbaren sowie wiederverschließbaren Verschließelement (4), welches für eine Probenprüfung durch eine Nadel in einer automatisierten und geschlossenen Betriebsart geeignet ist;
b) eine Leseregion (5) mit einem oder mehreren Reaktionspads (6), die in eine innere Wand des Behälters integriert sind, wobei jedes Reaktionspad (6) geeignet ist, eine vorbestimmte Menge von Urin zu absorbieren und eine kolorimetrische Reaktion auszulösen, die spezifisch für ein jeweiliges Analyt, einen Parameter und/oder eine Referenz ist;
c) ein Separationsseptum (10), das undurchlässig und durchdringbar ist sowie zwischen der Behältnisregion (6) und der Leseregion (5) angeordnet ist.

2. Behälter (1) nach Anspruch 1, wobei die Behältnisregion (9) ein vorkalibriertes Vakuum aufweist, welches geeignet ist, eine Menge von Urin aus einem primären Behälter einer gezogenen Probe neu abzurufen.

3. Behälter (1) nach Anspruch 1, wobei das Separationsseptum (10) wenigstens teilweise aus Aluminium gefertigt ist.

4. Behälter (1) nach Anspruch 1, wobei das Separationsseptum (10) eine Randregion (11) aus Kunststoffmaterial und eine durchdringbare Zentralregion (12) aus Aluminium umfasst.

5. Behälter (1) nach einem der vorhergehenden Ansprüche, wobei die innere Wand des Behälters wenigstens eine Vertiefungszone aufweist, die geeignet ist, ein jeweiliges Pad (6) in seiner ganzen Dicke zu enthalten.

6. Behälter (1) nach einem der vorhergehenden Ansprüche, wobei eines oder mehrere der Pads (6) aus einem Streifen (7) gefertigt sind, die an der inneren Wand der Leseregion (5) befestigt sind.

7. Behälter (1) nach Anspruch 6, wobei die innere Wand der Leseregion eine Vertiefungszone aufweist, die geeignet ist, den Streifen (7) in seiner ganze Dicke aufzunehmen.

8. Behälter (1) nach einem der vorhergehenden Ansprüche, wobei die Behältnisregion (9) und die Leseregion (5) wenigstens einen Teil einer planen Wand (15, 16, 17) aufweisen, wobei wenigstens ein Teil der planen Wand (15, 16, 17) konfiguriert ist, um die Reaktionspads (6) zu beherbergen.

9. Behälter (1) nach einem der vorhergehenden Ansprüche, wobei zu jedem Reaktionspad (6) ein Code (25) assoziiert ist, der ein korrespondierendes Analyt und/oder einen korrespondierenden Parameter identifiziert und der geeignet ist, um von einem optischen Lesegerät erkannt zu werden, das in den automatisierten Analysierer integriert ist.

10. Behälter (1) nach einem der vorhergehenden Ansprüche, wobei die Zonen zwischen verschiedenen Reaktionspads (6), die in der Leseregion (5) vorhanden sind, lichtdicht gefertigt sind.

11. Behälter (1) nach einem der vorhergehenden Ansprüche, welcher einen undurchlässigen Schutzfilm (30) umfasst, der anhaftend an der Rückseite der Reaktionspads (6) auf ihrer Seite, die der inneren Wand des Behälters (1) in der Leseregion (5) zugewandt ist, gefertigt ist, und der ein kalibriertes Loch (31) auf jeder Seite der Pads (6) aufweist, welches den Kontakt mit einer korrekten Menge von Urin erlaubt.

12. Verfahren zum Ausführen eines standardisierten vollständigen Tests von Urin in einem automatisierten Analysierer, einschließlich einer physikalisch-chemischen Überprüfung sowie von Harnsediment bezüglich Urin über einen automatisierten Analysierer in einer geschlossenen Betriebsart, welches die folgenden Verfahrensschritte aufweist:
a) Füllen eines Behälters (1) nach einem der vorhergehenden Ansprüche mit einer Urinprobe aus einem primären Aufnahmebehälter durch Saugen, welches durch ein vorkalibriertes Vakuum reguliert wird, in einer geschlossenen Betriebsart von außen;
b) Füllen des Behälters (1) an dem automatischen Analysierer;
c) Durchdringen des durchdringbaren und wiederverschließbaren Verschlusselementes (4) mit einer Proben- und Mischnadel (20), um so die Nadel (20) in die Behältnisregion (9) gemäß einer geschlossenen Betriebsart einzuführen;
d) Mischen der in der Behältnisregion (9) vorhandenen Probe und Herausziehen einer vorbestimmten Menge von Urin in einer geschlossenen Betriebsart;
e) Übertragen eine solchen vorbestimmten Menge zu dem Analysierer für das automatische Lesen von Harnsediment;
f) Durchdringen des Septums (10) und Zurückziehen der Probennadel auf eine solche Weise, dass der Urin in die Leseregion (5) fließt und in Kontakt mit den Reaktionspads (6) gelangt;
g) Warten über eine spezifizierte Zeit zur Erzeugung von Farbreaktionen;
h) Ausführen eines automatischen Lesens der kolorimetrischen Reaktionen unter Verwendung eines in dem Analysierer integrierten Sensors.

13. Verfahren nach Anspruch 12, wobei der Leseschritt eine Rotation des Behälters (1) entlang seiner Längsachse zur Verfügung stellt.

## Revendications

1. Récipient (1) apte à réaliser un test d'urine complet standard comprenant la détermination des analytes et/ou des paramètres physico-chimiques et de sédiment urinaire dans l'urine via un analyseur automatique en mode fermé, comprenant :
a) une région de confinement (9) ayant une ouverture (3) avec un élément de fermeture (4) de serrage et perforable et refermable, approprié pour prélever un échantillon grâce à une aiguille dans un mode automatique et fermé ;
b) une région de lecture (5) ayant un ou plusieurs tampons de réaction (6) rendus solidaire d'une paroi interne du récipient ; chaque tampon de réaction (6) étant apte à absorber une quantité d'urine préétablie et à donner lieu à une réaction colorimétrique spécifique pour un analyte, un paramètre et/ou référence respectif ;
c) un septum de séparation (10) imperméable et perforable, positionné entre ladite région de confinement (9) et ladite région de lecture (5).

2. Récipient (1) selon la revendication 1, dans lequel la région de confinement (9) a un vide pré-calibré approprié pour le rappel d'une quantité d'urine d'un récipient principal d'échantillon collecté.

3. Récipient (1) selon la revendication 1, dans lequel ledit septum de séparation (10) est réalisé, au moins en partie, à partir d'aluminium.

4. Récipient (1) selon la revendication 3, dans lequel ledit septum de séparation (10) comprend une région périphérique (11) en matière plastique et une région centrale perforable (12) en aluminium.

5. Récipient (1) selon l'une des revendications précédentes, dans lequel ladite paroi interne du récipient a au moins une zone d'évidement apte à contenir un tampon (6) respectif sur toute son épaisseur.

6. Récipient (1) selon l'une des revendications précédentes, dans lequel un ou plusieurs desdits tampons (6) est (sont) réalisé(s) sur une bande (7) fixée au niveau d'une paroi interne de ladite région de lecture (5).

7. Récipient (1) selon la revendication 6, dans lequel ladite paroi interne de ladite région de lecture (5) a une zone d'évidement apte à contenir ladite bande (7) sur toute son épaisseur.

8. Récipient (1) selon l'une des revendications précédentes, dans lequel ladite région de confinement (9) et ladite région de lecture (5) ont au moins une partie de paroi de plan (15, 16, 17), ladite au moins une partie de paroi de plan (15, 16, 17) étant configurée pour loger lesdits tampons de réaction (6).

9. Récipient (1) selon l'une des revendications précédentes, dans lequel à chaque tampon de réaction (6) est associé un code (25) identifiant un analyte et/ou un paramètre correspondant, apte à être reconnu par un lecteur optique intégré dans un analyseur automatique.

10. Récipient (1) selon l'une des revendications précédentes, dans lequel les zones entre les différents tampons de réaction (6) présents dans la région de lecture (5) sont rendues opaques.

11. Récipient (1) selon l'une des revendications précédentes, comprenant un film non perméable de protection (30) rendu adhérant à l'arrière desdits tampons de réaction (6) sur leur côté faisant face à la paroi interne du récipient (1) dans la région de lecture (5) et ayant un trou calibré (31) à chacun desdits tampons (6) permettant le contact avec une quantité correcte d'urine.

12. Procédé pour réaliser un test complet d'urine standard sur un analyseur automatique, comprenant l'examen physico-chimique et de sédiment urinaire dans l'urine via un analyseur automatique dans un mode fermé, qui comprend les étapes de procédure suivantes :
a) remplir un récipient (1) selon l'une quelconque des revendications précédentes avec un échantillon d'urine, à partir d'un récipient de collecte principal par aspiration régulé à partir d'un vide pré-calibré, en mode fermé depuis l'extérieur ;
b) charger le récipient (1) sur l'analyseur automatique ;
c) percer l'élément de fermeture (4) perforable et refermable avec une aiguille de prélèvement d'échantillon et de mélangeur (20) afin d'insérer l'aiguille (20) dans la région de confinement (9) selon un mode fermé ;
d) mélanger l'échantillon présent dans la région de confinement (9) et aspirer dans un mode fermé, une quantité prédéterminée d'urine ;
e) transférer une telle quantité prédéterminée dans l'analyseur pour la lecture automatique de sédiment urinaire ;
f) percer le septum (10) et rétracter l'aiguille de prélèvement d'échantillon (20) de sorte que l'urine s'écoule dans la région de lecture (5) venant en contact avec les tampons de réaction (6) ;
g) attendre une durée spécifiée pour la génération de réactions de couleur ;
h) réaliser une lecture automatique des réactions colorimétriques en utilisant un capteur intégré dans l'analyseur.

13. Procédé selon la revendication 12, dans lequel ladite étape de lecture fournit une rotation du récipient (1) le long de son axe longitudinal.
